# EUROPEAN PATENT APPLICATION

(11) **EP 4 198 884 A1**
(43) Date of publication of application: **21.06.2023**
(21) Application number: 21215884.4
(22) Date of filing: 20.12.2021
(51) Int. Cl.: G06T 7/00

(54) **METHOD AND SYSTEM FOR PROCESSING AN IMAGE**

(71) Applicant: Koninklijke Philips N.V., 5656 AG Eindhoven (NL)
(72) Inventor: LOSSAU, Tanja, Eindhoven (NL); KLINDER, Tobias, Eindhoven (NL); CAROLUS, Heike, Eindhoven (NL); WIENKER, Rafael, Eindhoven (NL); BROSCH, Tom, Eindhoven (NL)
(74) Representative: Philips Intellectual Property & Standards

(57) **Abstract**

According to an aspect, there is provided a computer implemented method of processing an image of a subject comprising lymph nodes, the method comprising: segmenting lymph nodes in image data corresponding to the image, delineating lymph nodes from the segmented image data, classifying a lymph node as belonging to a predefined region, evaluating a region of the image based on an assessment of the risk of the region comprising a malign lymph node, and indicating the evaluation of the region.

## Description

### FIELD OF THE INVENTION

The invention relates to methods of processing an image of a subject, and more particularly, methods of processing an image of a subject comprising lymph nodes.

### BACKGROUND OF THE INVENTION

TNM staging (T- tumor, N- lymph nodes, M- metastasis) based on Computed Tomography (CT) and/or Positron Emission Tomography (PET) imaging is a task in oncology applied at the time of diagnosis, after surgery, and in treatment response evaluation. The T stage is defined by the size and position of a primary tumour. The classification of the N and M stage requires examination of the spread of cancer into the lymphatic system on a regional basis.

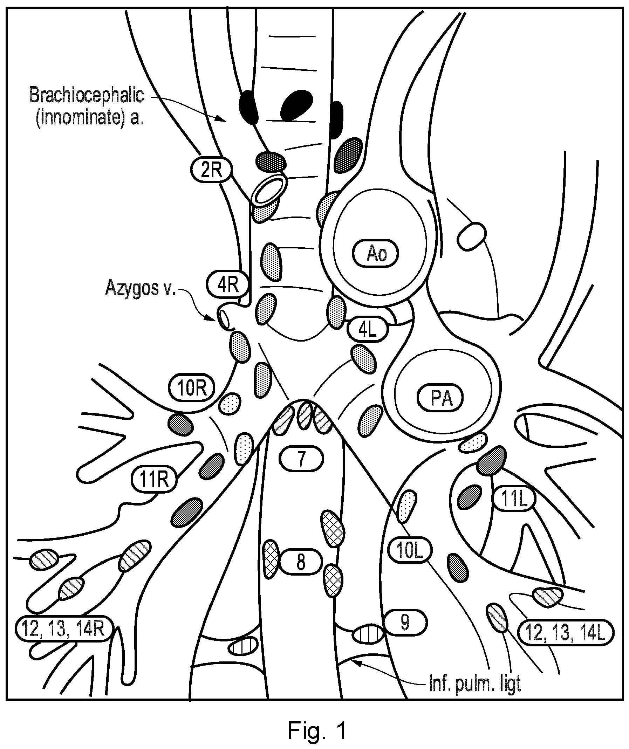

Affected lymph nodes can be identified in PET or CT images by assessing characteristics such as increased size, abnormal shape, inhomogeneous texture and high metabolic activity. However, the assessment of lymph nodes from PET or CT is an extremely challenging and time-consuming task due to (1) complex, cancer-specific labelling schemes such as visualized in Fig. 1, where lymph nodes belonging to different regions have different cross hatching (taken from Mountain, C. F., & Dresler, C. M. (1997). Regional lymph node classification for lung cancer staging. Chest, 111(6), 1718-1723., which illustrates an example of lymph node labelling in the mediastinum), (2) region-dependent risk boundaries, where risk indicators may differ depending on the region to which the lymph node belongs, (3) the large number of lymph nodes (about 300-700) widespread within the human body, (4) potentially unclear nodal boundaries resulting from small contrast differences between the lymph nodes and surrounding tissue or adjacent lymph nodes forming clusters. Although important for treatment decisions, clinical workflows typically used to assess lymph nodes, generally under high time pressure, only allow the assessment of a small set of a few selected lymph nodes, potentially leading to inaccurate staging results. Examples for such suboptimal staging results are, for example, missed enlarged lymph nodes, or missed lymph nodes that are not pathological in baseline but enlarge over time. For that reason, more advanced lymph node stratification is needed.

### SUMMARY OF THE INVENTION

Manual detection, measuring and labelling of potentially malignant lymph nodes in PET or CT images is time-consuming and strongly depends on the radiologists' experience due to the aforementioned challenges (1)-(4). As accurate delineation of lymph nodes can take several hours (especially in the case of lymphoma), assessment is limited to a few selected lymph nodes. Furthermore, a particular challenge is the assessment of lymphoma, where most lymph nodes increase in size and often grow into large bulks.

Rather than investigating a few selected lymph nodes only (as is usually done in the clinical workflow), the methods disclosed herein outline an approach for automatic risk assessment per nodal region in order to facilitate fast and accurate (T)NM staging and mitigate reader dependencies. Advantageously, region wise aggregations are less sensitive to slight inaccuracies for individual lymph nodes. The transition from node-by-node to region-wise lymphatic tissue node burden is further advantageous as the N and M stages are defined by nodal involvement on a regional basis, and therefore the results directly relate to these stages. Furthermore, such methods facilitate comparison of follow-up scans, as region wise assessment will likely be quicker and more accurate than attempting to find the same, previously assessed, lymph node for comparison.

According to an aspect, there is provided a computer implemented method of processing an image of a subject comprising lymph nodes, the method comprising: segmenting lymph nodes in image data corresponding to the image, delineating lymph nodes from the segmented image data, classifying a lymph node as belonging to a region, evaluating a region of the image based on an assessment of the risk of the region comprising a malign lymph node, and indicating the evaluation of the region. The region may be an anatomical region. The region of the image which is evaluated may correspond to, or be the same as, the region to which the lymph node has been classified.

Thus, a method is provided in which as assessment of the likelihood of regions comprising malignant lymph nodes is performed per region rather than per node. This advantageously improves the speed and accuracy with which an image of a subject can be assessed.

According to a further aspect, there is provided a method of training a machine learning model for use in processing an image of a subject comprising lymph nodes, the method comprising: obtaining training data, the training data comprising an image of a subject with at least one annotation of a lymph node, and the risk of the lymph node being malign or a set of rules for assessing whether a lymph node is malign, and an annotation indicating at least one region; and training the model based on the training data to classify a region of the image based on an assessment of the risk of the region comprising a malign lymph node.

According to a further aspect, there is provided a computer program product comprising a computer readable medium, the computer readable medium having computer readable code embodied therein, the computer readable code being configured such that, on execution by a suitable computer or processor, the computer or processor is caused to perform the methods described herein.

According to a further aspect, there is provided a system for processing an image of a subject comprising lymph nodes, the system comprising: a memory comprising instruction data representing a set of instructions; and a processor configured to communicate with the memory and to execute the set of instructions, wherein the set of instructions, when executed by the processor, cause the processor to: segment image data corresponding to the image, delineate lymph nodes from the segmented image data, classify a lymph node as belonging to a region, evaluating a region of the image based on an assessment of the risk of the region comprising a malign lymph node, and indicate the evaluation of the region.

These and other aspects will be apparent from and elucidated with reference to the embodiment(s) described hereinafter.

### BRIEF DESCRIPTION OF THE DRAWINGS

Example embodiments will now be described, by way of example only, with reference to the following drawings, in which:
Fig. 1 illustrates an example of lymph node labelling in the mediastinum;
Fig. 2 illustrates an example of a system according to an example;
Fig. 3 illustrates an example of a method according to an example;
Fig. 4 illustrates images involved in methods described herein;
Fig. 5 illustrates the images input and output in methods described herein; and
Fig. 6. illustrates images resulting from the methods described herein.

### DETAILED DESCRIPTION OF EMBODIMENTS

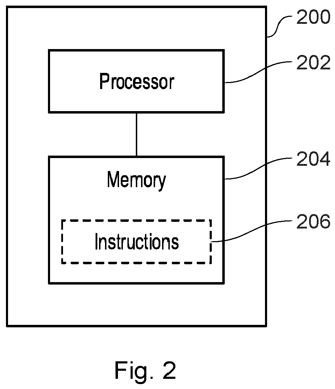

Turning now to Fig. 2 in some embodiments there is an apparatus 200 for use in method of processing an image of a subject comprising lymph nodes, according to some embodiments herein. Generally, the apparatus may form part of a computer apparatus or system e.g. such as a laptop, desktop computer or other computing device. In some embodiments, the apparatus 200 may form part of a distributed computing arrangement or the cloud.

The apparatus comprises a memory 204 comprising instruction data representing a set of instructions and a processor 202 (e.g. processing circuitry or logic) configured to communicate with the memory and to execute the set of instructions. Generally, the set of instructions, when executed by the processor, may cause the processor to perform any of the embodiments of the method of processing an image of a subject comprising lymph nodes as described below.

Embodiments of the apparatus 200 may be for use in processing an image of a subject comprising lymph nodes. More specifically, the set of instructions, when executed by the processor, cause the processor to: segment lymph nodes in image data corresponding to the image, delineate lymph nodes from the segmented image data, classify a lymph node as belonging to a region, evaluate a region of the image based on an assessment of the risk of the region comprising a malign lymph node, and indicate the evaluation of the region.

The processor 202 can comprise one or more processors, processing units, multi-core processors or modules that are configured or programmed to control the apparatus 200 in the manner described herein. In particular implementations, the processor 202 can comprise a plurality of software and/or hardware modules that are each configured to perform, or are for performing, individual or multiple steps of the method described herein. The processor 202 can comprise one or more processors, processing units, multi-core processors and/or modules that are configured or programmed to control the apparatus 200 in the manner described herein. In some implementations, for example, the processor 202 may comprise a plurality of (for example, interoperated) processors, processing units, multi-core processors and/or modules configured for distributed processing. It will be appreciated by a person skilled in the art that such processors, processing units, multi-core processors and/or modules may be located in different locations and may perform different steps and/or different parts of a single step of the method described herein.

The memory 204 is configured to store program code that can be executed by the processor 202 to perform the method described herein. Alternatively or in addition, one or more memories 204 may be external to (i.e. separate to or remote from) the apparatus 200. For example, one or more memories 204 may be part of another device. Memory 204 can be used to store the image, e.g. image of a subject, image data, segmented image data, results of the methods, the indication, images showing the indication, data, data indicating the evaluation, and/or any other information or data received, calculated or determined by the processor 202 of the apparatus 200 or from any interfaces, memories or devices that are external to the apparatus 200. The processor 202 may be configured to control the memory 204 to store the image, e.g. image of a subject, image data, segmented image data, results of the methods, the indication, images showing the indication, data, data indicating the evaluation, and/or any other information or data received, calculated or determined.

In some embodiments, the memory 204 may comprise a plurality of sub-memories, each sub-memory being capable of storing a piece of instruction data. For example, at least one sub-memory may store instruction data representing at least one instruction of the set of instructions, while at least one other sub-memory may store instruction data representing at least one other instruction of the set of instructions.

It will be appreciated that Fig. 2 only shows the components required to illustrate this aspect of the disclosure and, in a practical implementation, the apparatus 200 may comprise additional components to those shown. For example, the apparatus 200 may further comprise a display. A display may comprise, for example, a computer screen, and/or a screen on a mobile phone or tablet. The apparatus may further comprise a user input device, such as a keyboard, mouse or other input device that enables a user to interact with the apparatus, for example, to provide initial input parameters to be used in the method described herein. The apparatus 200 may comprise a battery or other power supply for powering the apparatus 200 or means for connecting the apparatus 200 to a mains power supply.

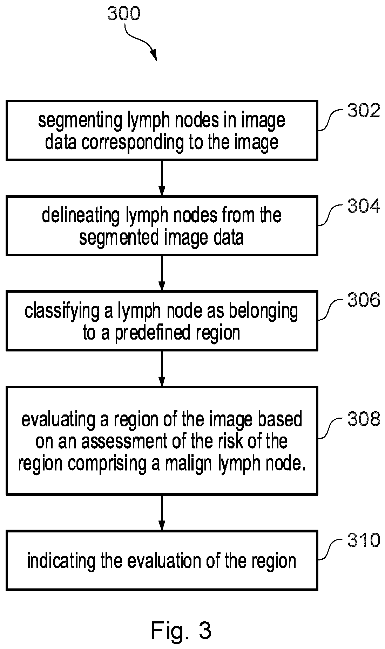

Turning to Fig. 3, there is a computer implemented method 300 for use in processing an image of a subject comprising lymph nodes. Embodiments of the method 300 may be performed, for example by an apparatus such as the apparatus 200 described above.

Briefly, in a first step 302, the method 300 comprises: segmenting lymph nodes in image data corresponding to the image. In a second step 304, the method comprises delineating lymph nodes from the segmented image data (e.g. separating individual or clusters of lymph nodes from segmented image data). In a third step 306, the method comprises classifying a lymph node as belonging to a region. In a fourth step 308, the method comprises evaluating a region of the image based on an assessment of the risk of the region comprising a malign lymph node. In a fifth step 310, the method comprises indicating the evaluation of the region.

The methods herein may be applied to a diverse range of images, such as for example, medical images. Image segmentation involves extracting shape/form information about the objects or shapes captured in an image. This may be achieved by converting the image into constituent blocks or "segments" that represent the different features in the image. In some methods, image segmentation may comprise fitting a model to one or more features in an image.

One method of image segmentation is Model-Based Segmentation (MBS), whereby a triangulated mesh of a target structure (such as, for example, a heart, brain, lung, lymph node etc.) is adapted in an iterative fashion to features in an image. Segmentation models typically encode population-based appearance features and shape information, and a model-based segmentation process may both segment and delineate (individual) lymph nodes in one step. Such information describes permitted shape variations based on real-life shapes of the target structure in members of the population. Shape variations may be encoded, for example, in the form of Eigenmodes which describe the manner in which changes to one part of a model are constrained, or dependent, on the shapes of other parts of a model.

Model-based segmentation has been used in various applications to segment one or multiple target organs from medical images, see for example, the paper by Ecabert, O., et al. 2008 entitled "Automatic Model-Based Segmentation of the Heart in CT Images"; IEEE Trans. Med. Imaging 27 (9), 1189-1201. The use of triangulated surface meshes has led to MBS generally providing smooth segmentation results. Furthermore, MBS is generally considered to be robust against image artifacts, such as variations in image quality.

Another segmentation method uses machine learning (ML) models to convert an image into a plurality of constituent shapes (e.g. block shapes or block volumes), based on similar pixel/voxel values and image gradients. This is described, for example, in the paper by Long et al. entitled: "Fully Convolutional Networks for Semantic Segmentation".

Voxel-wise segmentation (which may be performed by a voxel-wise segmentation network) may also be used to perform the methods described herein. In voxel-wise segmentation used in the methods described herein, for each voxel of the image data it may be determined whether the voxel belongs to a lymph node or not. The image data may first be segmented (e.g. to segment lymph nodes in the image data where for each voxel it is determined whether the voxel belong to a lymph node or node), and then (individual) lymph nodes may be delineated from the segmented image data (e.g. to determine the location of individual lymph nodes). Voxel -wise segmentation may be beneficial in the segmentation of lymph nodes as opposed to, for example, model-based segmentation due to the high variance of lymph node shapes.

Any appropriate segmentation method may be used to segment lymph nodes in an image. In more detail, the image (e.g. the image that is to be segmented) may be any type of image. In some embodiments, the image may comprise a scientific image. In some embodiments, for example, the image may comprise a medical image.

A medical image may comprise an image acquired using any imaging modality. Examples of a medical image include, but are not limited to, a computed tomography (CT) image (for example, from a CT scan) such as a C-arm CT image, a spectral CT image or a phase contrast CT Image, an x-ray image (for example, from an x-ray scan), a magnetic resonance (MR) image (for example, from an MR scan), an ultrasound (US) image (for example, from an ultrasound scan), fluoroscopy images, nuclear medicine images, or any other three dimensional medical image.

More generally, the image may comprise an image captured using a charged coupled device CCD, such as that in a camera. The skilled person will appreciate that embodiments herein may be applied to other types of images and/or other data sets that may be segmented.

Generally the image may comprise a two dimensional image or a three dimensional image. The image may comprise a plurality (or set) of image components. For example, in embodiments where the image comprises a two dimensional image, the image components may comprise pixels. In embodiments where the image comprises a three dimensional image, the three dimensional image, the image components may comprise voxels.

Features in the image may comprise any object (e.g. real or simulated), shape or part of an object or shape thereof that is visible (e.g. discernable) in the image. In embodiments where the image comprises a medical image, the feature may comprise an anatomical feature, or portion thereof, such as part of a lung, heart, brain, or any other anatomical feature. Lymph nodes may be comprised in the image.

The region may be evaluated with respect to at least one risk factor. A risk factor may comprise one of: volume of a lymph node; length of a short axis of a lymph node; length of a long axis of a lymph node; quantitative texture measure; Positron Emission Tomography, PET, intensity; an increased size of a lymph node; high metabolic activity; formation of a merged lymph node cluster; regional lymphatic tissue volume; grey-value statistics. The risk factor may be region dependent. For example, in some (anatomical) regions the size of lymph nodes may vary more than in others. An example of this are the lymph nodes below the ears where enlargement is not a sign of cancer. Thus, in an example, enlarged lymph nodes in one region will not be a risk factor, whereas enlarged lymph nodes in another region will be a risk factor. Risk factors in different regions may be give different weight.

The evaluation may comprise evaluating a plurality of lymph nodes with respect to at least one risk factor, and evaluating the region based on the evaluation of at least one of the plurality of lymph nodes corresponding to the region. A region may be an anatomical region, and/or may be a predefined region, where a region may be used in the method depending on the type of cancer to be assessed.

Indicating the evaluation may comprise outputting at least one of: a risk statistic of the region; a colour overlay corresponding to the image indicating the risk associated with the region; a heat map corresponding to the image indicating the risk associated with the region; an overlay on a potentially malignant lymph node.

The evaluating may further comprise comparing the assessment of risk of a region to a previously obtained assessment of risk of the region. The evaluating may further comprise determining if the region has at least one of: an increased risk compared to the previously assessed risk; high variation in the previous assessment of risk and the current assessment of risk compared to the previously assessed risk.

The evaluating may be performed for a plurality of regions of the image. The indication of the evaluation may comprise at least one of: region risk statistics; a list of regions with an indication of the classification of each region; a list of regions ordered based on the evaluation of each region; a heatmap corresponding to the evaluation of each region.

The method (or a part/parts of the method such as the evaluation and/or the classification) may be performed by a trained model. The model may comprise any type of model, such as, for example, a model trained using a machine learning process. Examples of models that may be used herein include, but are not limited to, neural network models such as deep learning neural networks, and random forest models.

The skilled person will be familiar with neural networks, but in brief, neural networks are a type of supervised machine learning model that can be trained to predict a desired output for given input data. Neural networks are trained by providing training data comprising example input data and the corresponding "correct" or ground truth outcome that is desired. Neural networks comprise a plurality of layers of neurons, each neuron representing a mathematical operation that is applied to the input data. The output of each layer in the neural network is fed into the next layer to produce an output. For each piece of training data, weights associated with the neurons are adjusted until the optimal weightings are found that produce predictions for the training examples that reflect the corresponding ground truths.

Although examples of neural networks are provided herein, the skilled person will appreciate that generally the trained model may comprise any model that may be trained to take as input an image product and output (e.g. predict) an indication of a shape descriptor for one or more features in the image, such as lymph nodes, a classification of a lymph node as belonging to a region, an evaluation of a region and so on.

In some embodiments, the product related to the image may comprise the image itself. For example, the image product related to the image may comprise a portion of the image (e.g. the whole or part of the original image). In other words, in some embodiments, a model may be used to predict shape descriptors for a feature in an image, based on the image itself (e.g. taking the image as input). In other examples, the image product may comprise a portion of the image (e.g. the whole or part of the original image) having undergone a pre-processing process. Examples of pre-processing processes include, but are not limited to, for example, smoothing or filtering the image in order to accentuate the feature (or smooth the background noise).

The trained model may be trained to perform the segmentation, delineation, classification and/or the evaluation based on training data comprising an image of a subject with at least one annotation of a lymph node, and the risk of the lymph node being malign or a set of rules for assessing whether a lymph node is malign, and an annotation indicating at least one region.

There may be further provided a method of training a machine learning model for use in processing an image of a subject comprising lymph nodes, the method comprising: obtaining training data, the training data comprising an image of a subject with at least one annotation of a lymph node, and the risk of the lymph node being malign or a set of rules for assessing whether a lymph node is malign, and an annotation indicating at least one region; and training the model based on the training data to evaluate a region of the image based on an assessment of the risk of the region comprising a malign lymph node.

There may be further provided a computer program product comprising a computer readable medium, the computer readable medium having computer readable code embodied therein, the computer readable code being configured such that, on execution by a suitable computer or processor, the computer or processor is caused to perform the methods described herein.

There may be further provided a system for processing an image of a subject comprising lymph nodes, the system comprising: a memory comprising instruction data representing a set of instructions; and a processor configured to communicate with the memory and to execute the set of instructions, wherein the set of instructions, when executed by the processor, cause the processor to: segment image data corresponding to the image, delineate lymph nodes from the segmented image data, classify a lymph node as belonging to a region, evaluate a region of the image based on an assessment of the risk of the region comprising a malign lymph node, and indicate the evaluation of the region.

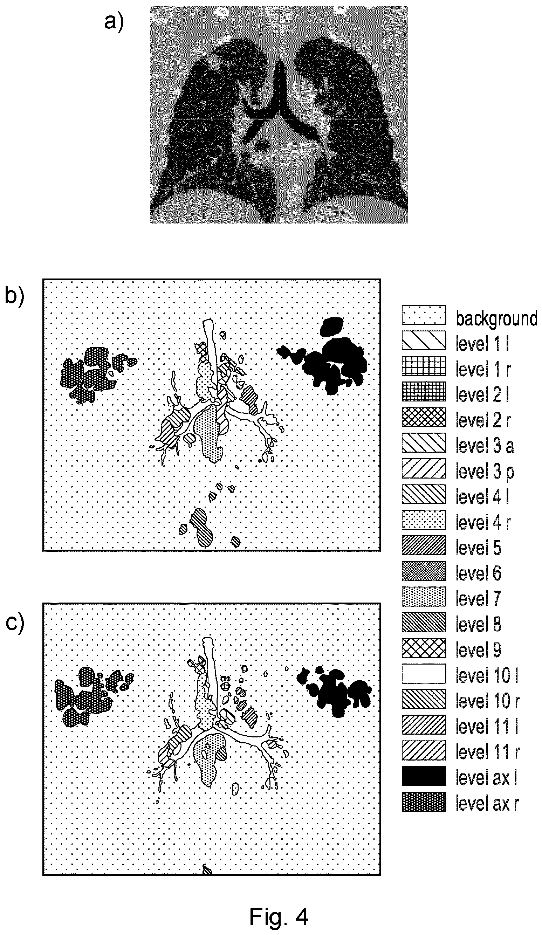

Thus, according to an example, the invention may use an image, such as volumetric PET and/or CT images, as primary input data. A first part of the method may comprise (automatic) lymph node segmentation, delineation and regional classification. Predictive models may be trained on a set of annotated data for the specific tasks as shown in Fig. 4.

Fig. 4 illustrates images involved in voxel-wise segmentation of lymph nodes as well as delineation and regional classification using a trained model. In this example, the model is trained in a data-driven way. The trained model takes a CT image volume as input and delivers a set of detected lymph nodes with associated voxel positions and the corresponding regional class of each lymph node as output (visualized as color-coded volume rendering, shown as differing cross hatching here). As is shown in this Fig., 4(a) illustrates an example of image data showing the chest area of a subject (e.g. an area comprising lymph nodes). 4(b) illustrates an example of the volume rendering of a target voxel mask (e.g. an annotated image of lymph nodes and corresponding regions, where different regions are illustrated in this Figure with different cross hatching). Fig. 4(c) shows an example of a rendering of the corresponding predicted mask generated in this example by a volumetric neural network (a neural network that has been trained using images such as the one of Fig. 4(b)). The key shown in Fig. 4 shows the different regions to which a lymph node can be classified as belonging (level 1r etc., for example corresponding to the indicated regions in Fig. 1) and the colour (shown here as cross hatching) associated with the respective regions.

As is discussed above, in a first part of the method, lymph nodes may be segmented from image data corresponding to an image, the lymph nodes may be delineated from the segmented image data, and lymph nodes may be classified as belonging to a region. The method may be performed by a trained model. One trained model may perform the whole method, or some parts of the method may be performed by one or more trained model, and other parts of the method may be performed by one or more trained model.

The trained model may be trained using a set of annotated training cases with pairs of CT image volumes of a subject/ subjects and corresponding lymph node (LN) annotations (see Fig. 4a,b). For example, the training data may comprise an image of a subject with at least one annotation of a lymph node, and the risk of the lymph node being malign or a set of rules for assessing whether a lymph node is malign, and an annotation indicating at least one region.

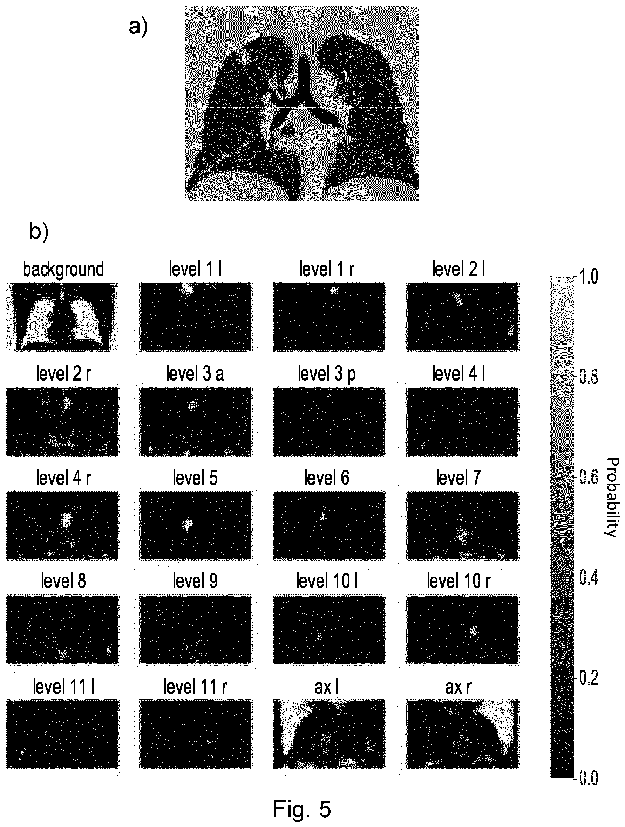

Using this training data, standard volumetric image segmentation or instance segmentation networks such as the foveal network (such as in Brosch, T., & Saalbach, A., 2018, March Foveal fully convolutional nets for multi-organ segmentation, In Medical Imaging 2018: Image Processing (Vol. 10574, p. 105740U) International Society for Optics and Photonics), or the Mask R-CNN (such as that shown in He, K., Gkioxari, G., Dollar, P., & Girshick, R. (2017), Mask R-CNN, In Proceedings of the IEEE international conference on computer vision (pp. 2961-2969)) are employed as predictors. The tasks of lymph node segmentation, delineation and regional classification can either be addressed by separate models or jointly trained via multi-task learning. The trained model or model ensemble may assign to each voxel within the image volume the probability of the voxel belonging to a certain lymph node and the probability of lying within a specific nodal region (see Fig. 5).

Fig. 5 illustrates a CT image volume (5(a)) which is used as input for a regional classification model, where the model then delivers a probabilistic mask as output (5(b)). Thus, for each region (e.g. "background" to "ax r" illustrated in Fig. 5, corresponding to the regions of Fig. 4) this Figure illustrates the probability of a voxel belonging to a certain lymph node as well as probability of the voxel lying within the region. The lymph node probability map may be thresholded to obtain a list of detected lymph nodes with associated voxel positions.

In a further part of the method, the method may comprise evaluating a region of the image based on an assessment of the risk of the region comprising a malign lymph node. In particular, each detected lymph node within a certain region may be evaluated with respect to at least one risk factor (e.g. risk feature) (e.g. volume of a lymph node; length of a short axis of a lymph node; length of a long axis of a lymph node; quantitative texture measure; Positron Emission Tomography, PET, intensity; an increased size of a lymph node; high metabolic activity; formation of a merged lymph node cluster; regional lymphatic tissue volume; grey-value statistics etc). The risk factor may be region dependent, whereby corresponding region-dependent risk boundaries may be taken into account. For example, corresponding region-dependent risk boundaries may be obtained from literature (or measurements on a cohort of healthy and non-healthy patients may be obtained) and stored within a dictionary, or look up table. For example, for lung cancer, Mountain, C. F., & Dresler, C. M. (1997). Regional lymph node classification for lung cancer staging. Chest, 111(6), 1718-1723 outlines regions for classification for lung cancer staging. The region(s) assessed may be predefined, and may depend on the type of cancer to be staged. Based on this dictionary and the risk factors, regional risk scores may be calculated for lymph nodes which have been determined as belonging to the region (e.g. based on a number of increased lymph nodes within region / number of metabolic active lymph nodes within region).The evaluating may further comprise comparing the assessment of risk of a region to a previously obtained assessment of risk of the region, for example to determine if the region has at least one of: an increased risk compared to the previously assessed risk; high variation in the previous assessment of risk and the current assessment of risk compared to the previously assessed risk.

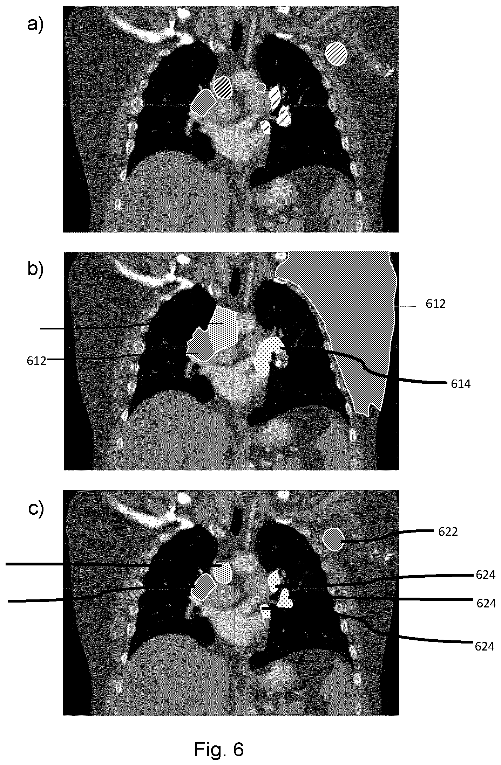

The evaluation of a region (or multiple regions) may be indicated (e.g. to the user). This may take the form of region-wise statistics (e.g. a risk statistic of the region, sorted lists starting with regions of increased risk and so on). For example, regional statistic of risk scores across multiple exams may be provided to a user in the form of sorted lists (e.g. starting with the region of highest risk or largest score changes compared to prior exams). Such regions may alternatively or additionally be highlighted in the CT image volume using an overlayed heatmap, or colour overlay, as shown in Fig. 6(b) (where different colours are here illustrated as different cross hatching).

Fig. 6 illustrates (a) detected lymph nodes which are assigned to regional classes (visualized via color coding, but shown as different cross hatched areas here, where the different cross hatching indicates the different regions to which the lymph nodes belong), where the individual lymph nodes are also evaluated with respect to region-dependent risk markers such as the nodal volume. As is illustrated in 6(b) a heatmap may be used to highlight regions of increased risk, for example where a region highlighted with the colour red 612 may denote 'high risk', orange 616 may denote 'medium risk' and yellow 614 may denote "low risk" (shown as differing cross hatching here), and so on, based on the assessment of the lymph nodes belonging to regions. Thus, a heatmap as visualized in Fig. 6(b) may indicate to the user critical image areas. In Fig. 6(c) particular lymph nodes within regions, which may be lymph nodes having particular influence on the risk level of a region, or any potentially malignant lymph node within a region, are highlighted (again, in this example, the lymph nodes are highlighted with their corresponding risk, where a lymph node highlighted with the colour red 622 may denote 'high risk', orange 626 may denote 'medium risk' and yellow 624 may denote "low risk" - in this example these colours are indicated by differing cross hatching). This enables easy review of particularly at risk lymph nodes by a user.

Thus, it is possible indicate to the user which areas of the image volume are of particular interest, and/or the order in which areas of the image volume should be considered (e.g. which areas of the image volume should be considered first). Furthermore, these methods may enable robust comparison of regional scores across multiple exams without the need for individual lymph node tracking.

Alternatively, a predictive model can be trained to combine the segmentation, delineation, classifying and evaluating to predict one joint risk score (e.g. the number of potentially malignant lymph nodes) per region.

An advantage associated with region based assessment is that the need for more complex lymph node wise tracking is negated by the region-based evaluation, and additionally progress monitoring with increased robustness regarding inaccuracies in lymph node segmentation is enabled. Furthermore, lymph nodes which are most influential in the regional risk decision can be displayed to the user (see Fig. 6(c)). The color-coded renderings (as depicted (as cross hatching) in Fig. 6, for example) may be linked to the original image volume, so that user-interaction such as a mouse-click on the colour coded volume rendering navigates to the corresponding location in a standard slice-wise viewport for further inspection, and vice versa. In case of (semi-automatic) adaptations of the lymph node mask, regional scores may be updated when the user is interacting with the image (or result, evaluation). For example, if the user is manually adding a lymph node or adjusting the shape in an existing delineation result, the steps of classifying a lymph node as belonging to a predefined region, evaluating a region of the image based on an assessment of the risk of the region comprising a malign lymph node, and indicating the evaluation of the region, may be repeated.

Particularly in a case of enlarged lymph nodes and merged lymph node clusters, a lymph node may touch multiple nodal regions. Advantageously, the methods described herein enable a regional probability mask to be used to calculate the contribution of a lymph node to multiple regions instead of assigning it to a specific regional class. Furthermore, if the position of the primary tumour is provided, N and M stage classification, which requires examination of the spread of cancer into the lymphatic system on a regional basis, may be enabled by the regional risk evaluation.

In another embodiment, there is provided a computer program product comprising a computer readable medium, the computer readable medium having computer readable code embodied therein, the computer readable code being configured such that, on execution by a suitable computer or processor, the computer or processor is caused to perform the method or methods described herein.

Thus, it will be appreciated that the disclosure also applies to computer programs, particularly computer programs on or in a carrier, adapted to put embodiments into practice. The program may be in the form of a source code, an object code, a code intermediate source and an object code such as in a partially compiled form, or in any other form suitable for use in the implementation of the method according to the embodiments described herein.

It will also be appreciated that such a program may have many different architectural designs. For example, a program code implementing the functionality of the method or system may be sub-divided into one or more sub-routines. Many different ways of distributing the functionality among these sub-routines will be apparent to the skilled person. The sub-routines may be stored together in one executable file to form a self-contained program. Such an executable file may comprise computer-executable instructions, for example, processor instructions and/or interpreter instructions (e.g. Java interpreter instructions). Alternatively, one or more or all of the sub-routines may be stored in at least one external library file and linked with a main program either statically or dynamically, e.g. at run-time. The main program contains at least one call to at least one of the sub-routines. The sub-routines may also comprise function calls to each other.

The carrier of a computer program may be any entity or device capable of carrying the program. For example, the carrier may include a data storage, such as a ROM, for example, a CD ROM or a semiconductor ROM, or a magnetic recording medium, for example, a hard disk. Furthermore, the carrier may be a transmissible carrier such as an electric or optical signal, which may be conveyed via electric or optical cable or by radio or other means. When the program is embodied in such a signal, the carrier may be constituted by such a cable or other device or means. Alternatively, the carrier may be an integrated circuit in which the program is embedded, the integrated circuit being adapted to perform, or used in the performance of, the relevant method.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A computer implemented method of processing an image of a subject comprising lymph nodes, the method comprising:
segmenting lymph nodes in image data corresponding to the image,
delineating lymph nodes from the segmented image data,
classifying a lymph node as belonging to a region,
evaluating a region of the image based on an assessment of the risk of the region comprising a malign lymph node, and
indicating the evaluation of the region.

2. The method as claimed in claim 1, wherein the region is evaluated with respect to at least one risk factor.

3. The method as claimed in claim 2, wherein a risk factor comprises one of: volume of a lymph node; length of a short axis of a lymph node; length of a long axis of a lymph node; quantitative texture measure; Positron Emission Tomography, PET, intensity; an increased size of a lymph node; high metabolic activity; formation of a merged lymph node cluster; regional lymphatic tissue volume; grey-value statistics.

4. The method as claimed in any preceding claim, wherein the evaluation comprises evaluating a plurality of lymph nodes with respect to at least one risk factor, and evaluating the region based on the evaluation of at least one of the plurality of lymph nodes corresponding to the region.

5. The method as claimed in any of claim 2 to 4, wherein the risk factor is region dependent.

6. The method as claimed in any preceding claim, wherein indicating the evaluation comprises outputting at least one of: a risk statistic of the region; a colour overlay corresponding to the image indicating the risk associated with the region; a heat map corresponding to the image indicating the risk associated with the region; an overlay on a potentially malignant lymph node.

7. The method as claimed in any preceding claim, wherein the evaluating further comprises comparing the assessment of risk of a region to a previously obtained assessment of risk of the region.

8. The method as claimed in claim 7, wherein the evaluating further comprises determining if the region has at least one of: an increased risk compared to the previously assessed risk; high variation in the previous assessment of risk and the current assessment of risk compared to the previously assessed risk.

9. The method as claimed in any preceding claim, wherein the evaluation is performed for a plurality of regions of the image.

10. The method as claimed in claim 9, wherein the indication of the evaluation comprises at least one of: region risk statistics; a list of regions with an indication of the evaluation of each region; a list of regions ordered based on the evaluation of each region; a heatmap corresponding to the evaluation of each region.

11. The method as claimed in any preceding claim, wherein the evaluation is performed by a trained model.

12. The method as claimed in claim 11, wherein the trained model has been trained to perform the evaluation based on training data comprising an image of a subject with at least one annotation of a lymph node, and the risk of the lymph node being malign or a set of rules for assessing whether a lymph node is malign, and an annotation indicating at least one region.

13. A method of training a machine learning model for use in processing an image of a subject comprising lymph nodes, the method comprising:
obtaining training data, the training data comprising an image of a subject with at least one annotation of a lymph node, and the risk of the lymph node being malign or a set of rules for assessing whether a lymph node is malign, and an annotation indicating at least one region; and
training the model based on the training data to evaluate a region of the image based on an assessment of the risk of the region comprising a malign lymph node.

14. A computer program product comprising a computer readable medium, the computer readable medium having computer readable code embodied therein, the computer readable code being configured such that, on execution by a suitable computer or processor, the computer or processor is caused to perform the method as claimed in any one of claims 1 to 13.

15. A system for processing an image of a subject comprising lymph nodes, the system comprising:
a memory comprising instruction data representing a set of instructions; and
a processor configured to communicate with the memory and to execute the set of instructions, wherein the set of instructions, when executed by the processor, cause the processor to:
segment lymph nodes in image data corresponding to the image,
delineate lymph nodes from the segmented image data,
classify a lymph node as belonging to a region,
evaluate a region of the image based on an assessment of the risk of the region comprising a malign lymph node, and
indicate the evaluation of the region.
